# EUROPEAN PATENT APPLICATION

(11) **EP 0 866 134 A2**
(43) Date of publication of application: **23.09.1998**
(21) Application number: 98103718.7
(22) Date of filing: 03.03.1998
(51) Int. Cl.: C12Q 1/52, C12Q 1/50, C12Q 1/32, C12Q 1/42, C12Q 1/48, C12Q 1/40, C12Q 1/46

(54) **A calibrator for measuring enzyme activity, and a method for measuring enzyme activity using the same**

(30) Priority: 17.03.1997 JP 62707/97
(71) Applicant: Hitachi, Ltd., Chiyoda-ku, Tokyo 101-0062 (JP)
(72) Inventor: Kondo, Shiho, Mito-shi, Ibaraki 310-0055 (JP); Imai, Kyoko, Hitachinaka-shi, Ibaraki 312-0033 (JP)
(74) Representative: von Hellfeld, Axel, Dr. Dipl.-Phys.

(57) **Abstract**

A calibrator comprising at least three kinds of enzymes having enzyme activity at least two times the upper reference limit of a reference interval for respective enzyme analysis items is disclosed, and calibration curves for respective of the enzyme analysis items are corrected with the calibrator. The calibrator can be used as a control sample.
According to the above procedure, apparatus differences of measured values can be reduced when an enzyme activity of a testing sample is measured.

## Description

### BACKGROUND OF THE INVENTION

The present invention relates to a preferable calibrator for measuring enzyme activity of a biological sample with an automatic analyzing apparatus, and a method for measuring enzyme activity using the same.

Clinical tests on various analysis items are indispensable in diagnosis of illness. In the clinical tests, the reliability of the measured values should be maintained at a high level. However, an interlaboratory difference, i.e. an inconsistency in measured values between the respective of the values measured at various testing laboratories on the same biological sample, actually exists. Main causes to generate the interlaboratory difference are assumed to be such as difference in the analytical apparatus used in respective of the testing laboratories (apparatus difference), difference in reagents used in respective of the testing (reagent difference), and so on.

Even in the testing of enzyme activity in biological samples such as blood and others, the problem of the interlaboratory difference is serious, and various attempts to overcome the problem have been performed. For instance, as a method for correcting the apparatus difference, a method to obtain a calibration coefficient (an observed K factor) specified to respective of the apparatus using a reaction indicating substance, of which concentration is previously known, is known as disclosed in "The Transaction for the Third Summer Seminar of the Japan Society for Clinical Chemistry", pages 68-71, (1983).

As a method for correcting the reagent difference, a method, wherein data output of a testing result is multiplied by a conversion coefficient of the reagent (KR value), is known. The KR value is determined based on a relationship between a reagent for standard measuring method and other respectively prepared reagents, which has been obtained previously by tests using many patient's samples. In accordance with the KR value, even if a test is performed with the respectively prepared reagents other than the reagents for the standard measuring method , the obtained result can be converted to the result measured by the standard measuring method. A result of studying the KR values of commercial reagents is disclosed in "The Medical Test", vol. 42, pages 1898∼1905, (1993).

Among the prior art described above, the method for correcting the apparatus difference requiring the observed K factor can calibrate a pipetting system and an optical system of the respective measuring apparatus. However, a measuring temperature is not included as a correcting factor. Therefore, the user of the measuring apparatus needed to confirm the temperature in the reaction bath with a thermistor thermometer, and if the temperature is shifted from the assumed measuring temperature, a correcting procedure was required.

As described above, in order to correct the interlaboratory difference in measuring the enzyme activity, determination of the observed K factor, confirmation of the measuring temperature, and data calibration with the KR value have been required at respective of the testing laboratories. The reason why such troublesome operations have been continued only in the enzyme items, and the calibration with a calibrator have not been generalized widely is because any superior enzyme sample for use as the calibrator has not been prepared. Furthermore, another reason is because the operation for preparing the calibrator has not been proceeded as expected on account of problems such as difficulties in a long term stability of the enzyme activity, in selection of an enzyme isozyme composition and an enzyme origin, which indicate a same reactivity with human blood serum, and others. However, currently, various enzyme calibrators became to be prepared successively, because the long term stability of the enzyme activity has come to be maintained by adding stabilizers or preservatives, and various problems relating to the enzyme isozyme composition and the enzyme origin have been resolved. Furthermore, because a JSCC (Japan Society for Clinical Chemistry) consensus method, i.e. a JSCC standard measuring method, for measuring the enzyme activity has been proposed by the Japan Society for Clinical Chemistry, a movement intending to unify the measured values by obtaining the measured values using the enzyme calibrator evaluated by the JSCC consensus method has been activated.

The activities of many commercially available enzyme calibrators are set at nearly an upper limit of the reference interval.

The reference interval means a concept, which come to be used in replacing a concept of a normal value range, indicating a reference value which is interchangeable and able to be shared with the respective laboratories based on the recommendation of the International Federation for Clinical Chemistry (IFCC). That is, taking a group of healthy people as a reference population, the reference interval is defined as an interval between the upper reference limit and the lower reference limit, wherein 95 % of the whole measured values are included in the range of the measured value distribution obtained by the measurement on respective of the person in the reference population.

At the field of clinical test, the enzyme calibrator having an activity near the upper reference limit is frequently used, because desirably the measurement is performed precisely in the vicinity of the reference interval in order to determine whether the data on a patient are normal or not. However, the apparatus can not be calibrated precisely depending on the above condition.

For instance, a case of ALT (alanine amino-transferase) is taken as an example, almost all the commercially available reagents for measuring ALT can measure at least approximately 1600 IU/liter. However, the ALT activity of the commercially available enzyme calibrator considering only the reference interval, i.e. 4∼44 IU/liter, of the JSCC consensus method, is approximately 50 IU/liter, which is equivalent to the variation in absorbance of -10 × 10⁻³ Abs./min. with the measuring wave length of 340 nm. Here, the IU means international unit.

Standard specification of a photometer enclosed in the automatic analyzing apparatus for measuring biological samples is approximately ± 1.0 × 10⁻³ Abs./min. In this case, it is assumed that if an output from the photometer fluctuates within the range of the specification by noise and the like in calibrating the calibration curve. That is, if the apparatus indicates - 11 × 10⁻³ Abs./min. when the calibrator should be measured as - 10 × 10⁻³ Abs./min., the apparatus has an error of 10 %. If the apparatus is used for measuring a biological sample with the calibrated condition as described above, the measured result has a possibility to generate a measuring error of 10 %. Accordingly, the measured results at respective laboratories fluctuate within the range of ± 10 %, such as the measured result at a laboratory includes -5 % error, and the measured result at another laboratory includes 9 % error. It means a large interlaboratory difference would be generated. Thus, the calibrator having a low activity is readily affected with an influence of error of the optical system in the apparatus, and the measuring error and the interlaboratory difference are readily generated.

Next, a case when ALT (Alanine aminotransferase) is measured as a sample for controlling accuracy using the commercially available calibrator (ALT activity 50 IU/liter) is considered.

The apparatus outputs the measured value as an integer by rounding off the small number less than the decimal point of the measured value to the nearest integer. Therefore, if the measured value is calculated in the apparatus as 49.5 IU/liter, the measured value is output as 50 IU/liter as expected, but if calculated as 50.5 IU/liter, it is output as 51 IU/liter, 1 IU/liter is shifted from the expected value. In either cases, the values calculated in the apparatus are shifted with a same ratio, 0.5 IU/liter, from the expected value. However, the final output results differ from each other, and only one of the results is output with a value shifted from the expected value by 2.0 % of error. Therefore, use of such calibration sample, which will be affected with an influence of error in the calculating process, is not preferable for maintaining the accuracy of the apparatus.

### SUMMARY OF THE INVENTION

The objects of the present invention is to provide a calibrator and a method for measuring enzyme activity , which can improve the measurement accuracy of an automatic analyzing apparatus when the enzyme activity is measured with, and decrease the difference in the measuring values in comparison with each of the analyzing apparatus from other analyzing apparatus.

The calibrator for measuring the enzyme activity relating to the present invention is characterized in containing at least three kinds of enzymes having enzyme activity, respectively, of at least two times of the upper reference limit of the reference interval for respective enzyme analysis item of objects, for which the calibration curves are prepared.

In the above case, the upper reference limits of the enzyme activities of the at least three kinds of enzymes are existed in the measurement effective range when analytical reagents corresponding to respective of the enzyme analysis items are used.

The enzymes contained in the calibrator are selected from the group consisting of Aspartate aminotransferase (AST), Alanine aminotransferase (ALT), Creatine knase (CK), Lactate dehydrogenase (LD), Alkaline phosphatase (ALP), γ-glutamyltransferase (GGT), Amylase (AMY), and Cholinesterase (CHE).

The calibrator for measuring enzyme activity based on the present invention is characterized in comprising at least three kinds of enzymes selected from the group consisting of Aspartate aminotransferase (AST), Alanine aminotransferase (ALT), Creatine knase (CK), Lactate dehydrogenase (LD), Alkaline phosphatase (ALP), and γ-glutamyltrans- ferase (GGT), and respective of the activities of the comprised enzymes is 250∼800 IU/liter for AST, 250∼800 IU/liter for ALT, 500∼2400 IU/liter for CK, 400∼800 IU/liter for LD, 700∼3200 IU/liter for ALP, and 200∼1000 IU/liter for GGT.

The method for measuring enzyme activity based on the present invention comprising the steps of measuring variation of absorbance of the reactant relating to the enzyme analysis items of the object sample, and determining the enzyme activity of the object sample based on the calibration curve, which has been prepared using the calibrator for measuring enzyme activity, characterized in preparing the calibration curve for respective of the analysis items corresponding to each of the at least three kinds of enzymes using the calibrator containing at least three kinds of enzymes, of which respective activity is of at least two times of the upper reference limit of the reference interval for respective enzyme analysis items.

### BRIEF DESCRIPTION OF THE DRAWINGS

These and other objects, features and advantages of the present invention will be understood more clearly from the following detailed description with reference to the accompanying drawings, wherein,
FIG. 1 is a schematic perspective view illustrating an overall composition of automatic analyzing apparatus relating to the present invention, and
FIG. 2 is a graph indicating an example of the calibration curve prepared by the method of the present invention.

### DESCRIPTION OF THE PREFERRED EMBODIMENTS

The enzyme used as the calibrator for measuring enzyme activity is desirably originated from any of human cells, human organs, animal cells, animal organs, and microorganisms. The enzyme calibrator is provided in a form of solution, which is prepared so that a specific gravity and a viscosity of the final solution are as approximately same as that of pooled serum by adding any of human pooled serum prepared by collecting human serum, human serum processed by degreasing, or 6 % bovine serum albumine solution.

The calibrator in accordance with the present invention contains at least three kinds of enzymes, preferably six kinds of enzymes, selected from the group consisting of Aspartate aminotransferase (AST), Alanine aminotransferase (ALT), Creatine knase (CK), Lactate dehydrogenase (LD), Alkaline phosphatase (ALP), γ-glutamyltransferase (GGT), Amylase (AMY), and Cholinesterase (CHE). The enzyme activity in the calibrator is at least two times of the upper reference limit of the reference interval for respective of the analysis items (inspection object items), and is adjusted to be in the range, which is less than the maximum value of the measurable limit based on the JSCC consensus method. The calibration curves for plural enzyme analysis items are prepared by a single calibrator.

In accordance with the JSCC consensus method, the enzyme activities of the calibrators based on the present invention are 250∼800 IU/liter for AST, 250∼800 IU/liter for ALT, 500∼2400 IU/liter for CK, 400∼800 IU/liter for LD, 700∼3200 IU/liter for ALP, and 200∼1000 IU/liter for GGT. The enzyme standard samples such as above can be used as control samples in addition to the calibrators.

Hereinafter, the embodiments of the present invention are explained in detail.

### (1) Preparation of the enzyme calibrator for preparing calibration curves

A phosphoric acid buffer solution (pH = 7.0) of 0.2 mol/liter is prepared. Sodium chloride, sodium azide, bovine serum albumine, pyridoxal phosphate, N-acetyl L-cysteine (NAC), ethylenediaminetetraacetic acid (EDTA), and magnesium chloride are dissolved into the phosphoric acid buffer solution. Concentration of each of the additives is adjusted to be 0.9 wt. % for sodium chloride, 2 mmol/liter for sodium azide, 6 wt. % for bovine serum albumine, 0.5 mmol/liter for pyridoxal phosphate, 15 mmol/liter for N-acetyl L-cysteine (NAC), 1 mmol/liter for ethylenediaminetetraacetic acid (EDTA), and 1.5 mmol/liter for magnesium chloride.

AST, ALT, CK, LD, ALP, and GGT made by Sigma Company are dissolved into the phosphoric acid buffer solution containing the above additives. Each of the above enzymes is added into the solution so that the activity of respective enzyme becomes 250∼800 IU/liter for AST, 250 ∼800 IU/liter for ALT, 500∼2400 IU/liter for CK, 400∼ 800 IU/liter for LD, 700∼3200 IU/liter for ALP, and 200 ∼1000 IU/liter for GGT, when the activity of the sample is determined by the JSCC consensus method.

The above calibrator is an example containing six kinds of enzymes. However, in accordance with preparing a calibrator containing at least three kinds of enzymes, the calibrator can be utilized for preparing calibration curves of at least three analysis items by only setting the calibrator into an automatic analyzing apparatus. As the three kinds of enzymes, AST, ALT, and LD are selected for example, and the calibrator is prepared by the same method as the above example. However, the combination of enzymes is not restricted to the above example.

The values of disclosed reference intervals are 8∼ 38 IU/liter for AST, 4∼44 IU/liter for ALT, 29∼203 IU/liter for children (C) of CK, 29∼145 IU/liter for female (F) of CK, 58∼348 IU/liter for male (M) of CK, 106∼211 IU/liter for LD, 104∼338 IU/liter for ALP, 5∼50 IU/liter for female (F) of GGT, and 5∼83 IU/liter for male (M) of GGT. With the above indication of the ranges, the prior number indicates the minimum reference limit, and the rear number indicates the maximum reference limit of the reference interval. The calibrator based on the present invention is prepared so that the enzyme activity indicates substantially more than two times of the upper reference limit of the reference range for respective enzyme analysis item.

The enzyme activity of the prepared enzyme samples is measured and designated its value in accordance with the JSCC consensus method. The enzyme samples are contained in a container of a predetermined capacity, the container is capped with a lid and sealed, and a label indicating the designated value of the respective enzyme activity is adhered to the outer wall of the container. The designated value is deemed as the known activity of the respective enzyme.

### (2) An example of preparing the calibration curve of the enzyme analysis items by an automatic analyzing apparatus

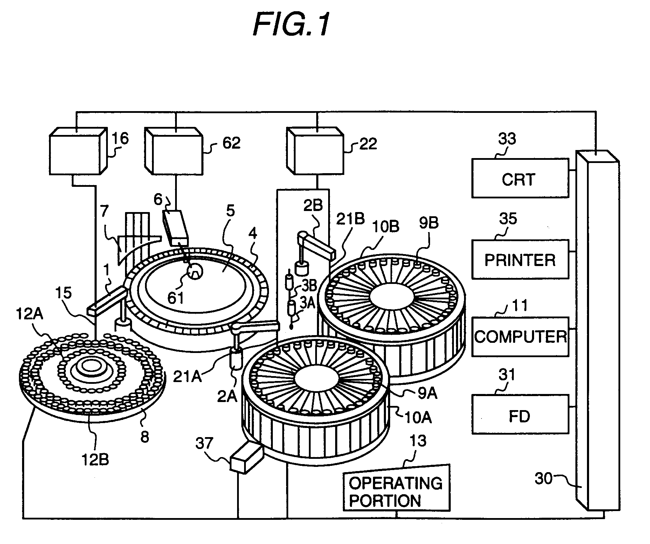

The enzyme calibrator prepared as above is used for calibrating the automatic analyzing apparatus as indicated in FIG. 1.

First, an example of the composition of the automatic analyzing apparatus is explained hereinafter. In accordance with FIG. 1, a plurality of sample containers 12B, which contain testing samples composed of biological samples such as serum, are arranged circularly at the outer periphery on a sample disk 8; and a plurality of the sample containers 12A, which contain reference samples for various analysis items and the enzyme calibrator, are arranged circularly at the inner periphery on the sample disk 8. A row of reaction vessel 4 for reacting the samples and reagents are arranged circularly on a reaction disk 5, which forms a reaction line. Reagent bottles 9A containing first reagents corresponding to plural analysis items are arranged on a reagent disk 10A for the first reagent, and reagent bottles 9B containing second reagents corresponding to plural analysis items are arranged on the reagent disk 10B for the second reagent.

Sample pipetting nozzle 15, which is attached to a movable arm of a sample pipetting mechanism 1, is connected to a syringe pump 16 for the sample. Reagent pipetting nozzle 21A, which is attached to a movable arm of a first reagent pipetting mechanism 2A; and reagent pipetting nozzle 21B, which is attached to a movable arm of a second reagent pipetting mechanism 2B, are connected to a syringe pump 22 for the reagent. A light source 61 for white light and a multi-wavelength photometer 6 are attached to the reaction disk 5, and the row of the reaction vessels 4 traverses a light flux from the light source 61. Data measured by the photometer 6 are converted by an analog-digital converter 62, and are input into a microcomputer 11 of a controlling part via an interface 30. Operation of respective mechanisms in the automatic analyzing apparatus are controlled by the microcomputer 11. Control programs are stored in floppy disks 31 of external memories.

Analysis parameters necessary for performing the analyzing operation are input from an operation part 13, and indicated in CRT 33 of an image display apparatus. The sample containers 12A of the enzyme calibrators, which are prepared in accordance with the above operation (1), are set on the sample disk 8; the analysis items for which the calibration curves to be prepared are input from the operation part 13 such as AST, ALT, CK, LD, ALP, and GGT; and the known activity of the respective of the enzymes in the calibrator is input from the operation part 13. The microcomputer 11 stores these data into the memories correlating to the setting position of the sample containers 12A on the sample disk 8. Although only one calibrator is sufficient for preparing the calibration curves, plural calibrators having different enzyme activities from each other can be used depending on necessity. Labels indicating reagent information are adhered onto the outer wall of the reagent bottles 9A, 9B, which are necessary for reactions of the respective analysis items of AST, ALT, CK, LD, ALP, and GGT on the reagent disk 10A, 10B; the reagent information are read out from the labels by an identification reader 37 ( for instance, a bar-code reader); and the respective of the reagent information is stored into the memories of the microcomputer 11 correlating with the setting position of the respective of the reagents on the reagent disk.

When the sample disk 8 is rotated and the sample container 12A containing the enzyme calibrator is positioned at a sample taking position, the sample pipetting mechanism 1 takes a predetermined amount of enzyme calibrator for AST analysis into the nozzle 15 from the sample container 12A, and delivers into one of the reaction vessels 4 on the reaction disk 5. Then, the reaction disk is rotated; the sample pipetting mechanism 1 delivers the same enzyme calibrator into the next reaction vessel 4 for ALT analysis as same as the above operation; and the sample pipetting mechanism 1 delivers the same enzyme calibrator into the next reaction vessel 4 for LD analysis as same as the above operation. In accordance with the same operation, the same calibrator is delivered to respective reaction vessels for other three enzyme items. The reaction disk 5 is rotated by one rotation and a distance equivalent to one vessel per every delivery of the calibrator.

When the respective reaction vessels 4 corresponding to the respective enzyme analysis items are stopped temporarily at the first reagent pipetting position and the second reagent pipetting position in accordance with subsequently repeating the intermittent rotation of the reaction disk 5, the reagents corresponding to the respective analysis items are delivered to the respective reaction vessels 4 by the reagent pipetting mechanism 2A, 2B, and the reactions corresponding to the respective analysis items occur in the reaction vessels. The mixed reactants in the respective reaction vessels after delivering the reagents are stirred by stirring mechanism 3A, 3B. The reactant in the reaction vessel is measured photometrically when a plurality of the reaction vessels traverse the light flux from the photometer 6, and the photometrical signals are converted to degrees of light absorbance. With respect to the respective enzyme analysis items of the enzyme calibrator, the variation in the degree of the absorbance are calculated by the microcomputer 11 from plural photometrical data obtained by traversing the light flux plural times with the reactants in the same reaction vessel during the enzyme reactions. And the calibration curve for respective analyst item of the AST, ALT, CK, LD, ALP, and GGT is calculated based on a relationship between the known activity of the respective enzymes and the obtained variation in the degree of the absorbance.

Then, the reagents corresponding to the analysis items are pipetted to the testing samples, which have been delivered respectively from the sample containers 12B to the reaction vessels 4, by the sample pipetting mechanism 1; the reactants are measured photometrically; the variation in the degree of the absorbance of the testing sample is obtained; and the enzyme activity in the testing sample for the respective of the analysis items is calculated by the microcomputer 11 based on the calibration curve, which has been prepared previously. The calculated enzyme activity as a measured value (an analytical result) for the testing sample is displayed in CRT 33 and the printer 35, respectively, correlating to the respective of the testing sample numbers and the analysis items. The reaction vessels 4 after finishing the photometrical measurement are washed by a washing mechanism 7, and are re-used for the analysis of the subsequent samples.

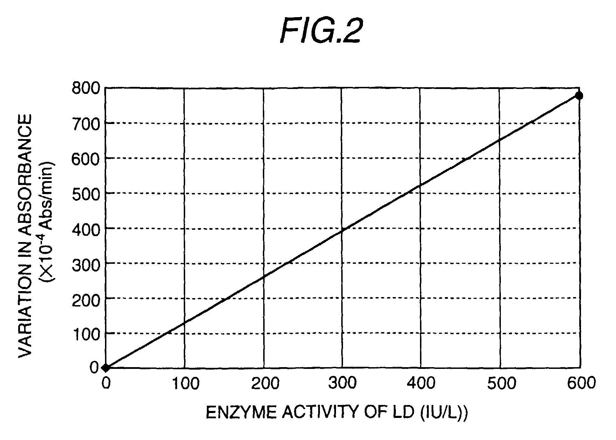

FIG. 2 is a graph indicating an example of the calibration curve prepared by the operation described above. The abscissa indicates the enzyme activity and the ordinate indicates the variation in the degree of absorbance. The example shown in FIG. 2 is the calibration curve for LD obtained from an observed value on the calibrator containing LD, of which enzyme activity is 600 IU/liter, and an observed value on a blank sample. Same linear calibration curves can be obtained on other five kinds of enzymes using the same calibrator.

The enzyme calibrator such as explained above can be used as a sample for controlling the accuracy of analyzing apparatus. The accuracy of the analyzing apparatus can be controlled by measuring the enzyme calibrator with a blank sample after analyzing testing samples for every predetermined times, for instance, every 300 testing samples measurement, or for every predetermined time, for instance, every two hours.

### (3) Estimation of the apparatus difference

Influence of the enzyme calibrator to a measurement error was studied with a commercial calibrator having a LD activity of 200 IU/liter and the enzyme calibrator (LD activity is approximately 600 IU/liter) prepared by the method described in the above item (1). Specified value for a noise level of the variation in the degree of absorbance of the photometer in the automatic analyzing apparatus is postulated to be within ± 1.0 × 10⁻³ Abs./min. In accordance with the commercial calibrator, the variation in the degree of the absorbance of the LD activity 200 IU/liter is equivalent to 26 × 10⁻³ Abs./min. when the noise level of the photometer is approximately zero. However, when the noise of the photometer influences within its specified value, the variation in the degree of the absorbance of the calibrator can be in the range of 25 × 10⁻³ ∼27 × 10⁻³ Abs./min. As a result, a measurement error in the range of -3.8 ∼ +3.8 % can be generated.

If the same calibration using the commercial enzyme calibrator is assumed to be performed with two automatic analyzing apparatus, the maximum apparatus difference can be 7.6 %. On the other hand, in accordance with the enzyme calibrator of the present invention, the variation in the degree of the absorbance is 78 × 10⁻³ Abs./min. When the noise of the photometer influences within its specified value, the variation in the degree of the absorbance of the calibrator can be in the range of 77 × 10⁻³ ∼79 × 10⁻³ Abs./min. The range is equivalent to a measurement error in the range of -1.3 ∼ +1.3 %, and the apparatus difference can be reduced to 2.6 % at maximum.

Similarly, the apparatus difference with AST and ALT are considered.

The AST activity of the commercial calibrator is 100 IU/liter, and the variation in the degree of the absorbance of the calibrator is - 20 × 10⁻³ Abs./min. When the noise of the photometer (± 1.0 × 10⁻³ Abs./min.) influences, the variation in the degree of the absorbance of the calibrator becomes in the range of -21 × 10⁻³ ∼ -19 × 10⁻³ Abs./min., and a measurement error in the range of -4.8 ∼ +5.3 % is generated. On the other hand, the AST activity of the enzyme calibrator of the present invention is 300 IU/liter, and the variation in the degree of the absorbance is -60 × 10⁻³ Abs./min. When the noise of the photometer (± 1.0 × 10⁻³ Abs./min.) influences, the variation in the degree of the absorbance is to be in the range of -61 × 10⁻³ ∼-59 × 10⁻³ Abs./min. The range is equivalent to a measurement error in the range of -1.6 ∼ +1.7 %, and the apparatus difference can be reduced to approximately 1/3 of the commercial calibrator.

The ALT activity of the commercial calibrator is 80 IU/liter, and the variation in the degree of the absorbance of the calibrator is - 16 × 10⁻³ Abs./min. When the noise of the photometer (± 1.0 × 10⁻³ Abs./min.) influences, the variation in the degree of the absorbance of the calibrator becomes in the range of -17 × 10⁻³ ∼ -15 × 10⁻³ Abs./min., and a measurement error in the range of -5.9 ∼ +6.7 % is generated. On the other hand, the ALT activity of the enzyme calibrator of the present invention is 250 IU/liter, and the variation in the degree of the absorbance is -50 × 10⁻³ Abs./min. When the noise of the photometer (± 1.0 × 10⁻³ Abs. /min.) influences, the variation in the degree of the absorbance is to be in the range of -51 × 10⁻³ ∼-49 × 10⁻³ Abs./min. The range is equivalent to a measurement error in the range of -2.0 ∼ +2.0 %, and the apparatus difference can be reduced to approximately 1/3 of the commercial calibrator.

In accordance with the calibration using the enzyme calibrator based on the present invention, the analyzing apparatus can be calibrated precisely, and the apparatus difference can be reduced.

### (4) Experimental results

Effects of correcting the apparatus difference were studied by making a survey using the enzyme calibrator based on the present invention. The number of the analyzing apparatus surveyed was 70, and the survey was performed on seven kinds of the analyzing apparatus such as HITACHI 7150 Type, HITACHI 7070 Type, HITACHI 7170 Type, HITACHI 7020 Type, HITACHI 7250 Type, HITACHI 7350 Type, and HITACHI 7450 Type.

The samples for the survey are the enzyme calibrator based on the present invention, a calibrator made by A company, and a human pooled serum as a testing sample. In accordance with the JSCC consensus method, the enzyme activities of the calibrators based on the present invention are AST (420 IU/liter), ALT (288 IU/liter), LD (673 IU/liter), CK (654 IU/liter), ALP (801 IU/liter), and GGT (440 IU/liter). On the other hand, the enzyme activities of the calibrator made by A company in accordance with the JSCC consensus method are AST (103 IU/liter), ALT (83 IU/liter), LD (258 IU/liter), CK (272 IU/liter), ALP (459 IU/liter), and GGT (154 IU/liter). With 70 automatic analyzing apparatus installed in various clinical testing laboratories separately, the human pooled serum was measured as the testing sample using respective of the enzyme calibrator based on the present invention and the samples made by the A company as the calibrator. And, convergence of the apparatus difference in the measurement of the human pooled serum using respective of the above two calibrators was compared each other. As the reagents for the measurement, the reagents for daily routine use in the respective laboratories were used.

The results of the survey are indicated in Table 1. The data exceeding the average value ± 2.7 SD were previously discarded. The apparatus difference can be judged from CV (%). The CV (%) indicates a degree of fluctuation in data. The smaller the CV (%) is, the smaller are the fluctuation of data in all the laboratories, and the apparatus difference can be said to be converged. As Table 1 reveals, the apparatus difference obtained using the enzyme calibrator based on the present invention is smaller than the apparatus difference obtained using the commercial calibrator. The N means the number of the apparatus.

**Table 1**

| | AST | | ALT | | LD | |
|---|---|---|---|---|---|---|
| Calibrator | Present invention | Made by A Co | Present invention | Made by A Co. | Present invention | Made by A Co. |
| MEAN (IU/L) | 173 | 170 | 70 | 68 | 356 | 352 |
| SD | 1.56 | 5.01 | 1.40 | 2.16 | 6.05 | 10.9 |
| CV (%) | 0.9 | 2.9 | 2.0 | 3.2 | 1.7 | 3.1 |
| N | 70 | 70 | 70 | 67 | 70 | 69 |

| | CK | | ALP | | GGT | |
|---|---|---|---|---|---|---|
| Calibrator | Present invention | Made by A Co | Present invention | Made by A Co. | Present invention | Made by A Co. |
| MEAN (IU/L) | 482 | 519 | 528 | 671 | 205 | 217 |
| SD | 7.23 | 15.1 | 10.8 | 25.0 | 6.15 | 9.11 |
| CV (%) | 1.5 | 2.9 | 2.0 | 3.7 | 3.0 | 4.2 |
| N | 70 | 70 | 65 | 65 | 61 | 61 |

### (5) Accuracy control

Accuracy control was performed using the enzyme calibrator, which was in the same lot as the sample used for the survey tests described in the above item (4), and the calibrator sample made by the A company as control samples, and measuring the samples twice a day for 20 days. Commercial JSCC standard reagents were used as the reagents for the measurement.

The results are indicated in Table 2. The measuring error could be retained smaller, and the fluctuation in all the six enzymes were smaller when using the calibrator based on the present invention than the case when the calibrator made by the A company was used.

In accordance with the present invention, the calibration curves for automatic analyzing apparatus can be prepared using the calibrator containing at least three kinds of enzymes so as to be at least two times of the upper reference limit of the reference interval. Therefore, the measuring accuracy of the automatic analyzing apparatus in measurement of enzyme activity of biological samples can be improved, and the measuring differencies among mutually different apparatus can be reduced.

**Table 2**

| | AST | | ALT | | LD | |
|---|---|---|---|---|---|---|
| Control | Present invention | Made by A Co | Present invention | Made by A Co. | Present invention | Made by A Co. |
| MEAN (IU/L) | 432 | 101 | 300 | 79 | 660 | 245 |
| SD | 7.8 | 3.0 | 6.0 | 2.5 | 14.5 | 6.9 |
| CV (%) | 1.8 | 3.0 | 2.0 | 3.2 | 2.2 | 2.8 |

| | CK | | ALP | | GGT | |
|---|---|---|---|---|---|---|
| Control | Present invention | Made by A Co | Present invention | Made by A Co. | Present invention | Made by A Co. |
| MEAN (IU/L) | 613 | 249 | 766 | 414 | 415 | 133 |
| SD | 15.3 | 7.47 | 23.0 | 14.5 | 8.7 | 3.46 |
| CV (%) | 2.5 | 3.0 | 3.0 | 3.5 | 2.1 | 2.6 |

## Claims

1. A calibrator for measuring enzyme activity comprising an enzyme, of which activity is known, further comprising:
at least three kinds of enzymes, of which the respective enzyme activity is at least two times of the upper reference limit of a reference interval for respective enzyme analysis items of a calibration curve preparing object.

2. The calibrator for measuring enzyme activity as claimed in claim 1, wherein
said upper reference limit of activities of said at least three kinds of enzymes are within a measurement effective range of analytical reagents corresponding to said respective of enzyme analysis items.

3. The calibrator for measuring enzyme activity as claimed in claims 1 or 2, wherein
said at least three kinds of enzymes are selected from the group consisting of Aspartate aminotransferase (AST), Alanine aminotransferase (ALT), Creatine knase (CK), Lactate dehydrogenase (LD), Alkaline phosphatase (ALP), γ-glutamyltransferase (GGT), Amylase (AMY), and Cholinesterase (CHE).

4. A calibrator for measuring enzyme activity comprising an enzyme, of which activity is known, further comprising:
at least three kinds of enzymes, which are selected from the group consisting of Aspartate aminotransferase (AST), Alanine aminotransferase (ALT), Creatine knase (CK), Lactate dehydrogenase (LD), Alkaline phosphatase (ALP), γ-glutamyltransferase (GGT), Amylase (AMY), and Cholinesterase (CHE), respective of the activity of which is 250∼800 IU/liter for AST, 250∼800 IU/liter for ALT, 500∼2400 IU/liter for CK, 400∼800 IU/liter for LD, 700∼3200 IU/liter for ALP, and 200∼1000 IU/liter for GGT.

5. A method for measuring enzyme activity comprising the steps of:
measuring variation in a degree of absorbance of reactant relating to enzyme analysis items of a testing sample, and said enzyme activity of said testing sample is determined on the basis of a calibration curve, which has been prepared previously using a calibrator for measuring enzyme activity, wherein said calibration curve for analysis items corresponding to respective of at least three kinds of enzymes is prepared using a calibrator comprising
said at least three kinds of enzymes having the enzyme activity at least two times of the upper reference limit of a reference interval for respective of the enzyme analysis items.
